# EUROPEAN PATENT APPLICATION

(11) **EP 0 552 593 A1**
(43) Date of publication of application: **28.07.1993**
(21) Application number: 92830640.6
(22) Date of filing: 25.11.1992
(51) Int. Cl.: A61L 27/00, A61L 31/00, A61K 31/73

(54) **Use of hyaluronic acid as ocular prosthesis lubricant**

(30) Priority: 20.01.1992 IT RM920039
(71) Applicant: SOCIETA'INDUSTRIA FRAMACEUTICA ITALIANA S.p.A., I-95128 Catania (IT)
(72) Inventor: Santocono, Marcello, c/o Societa Industria Farm., I-95128 Catania (IT); Ceccarini, Constante, c/o Societa Industria Farm., I-95128 Catania (IT); Stagni, Marcello, c/o Societa Industria Farm., I-95128 Catania (IT); Benanti, Giuseppe, c/o Societa Industria Farm., I-95128 Catania (IT)
(74) Representative: de Simone, Domenico

(57) **Abstract**

The use of compositions comprising hyaluronic acid is described to lubricate a substitutive prosthesis of the ocular bulb to be fitted to sub-atrophic or microophtalmic bulbs of eyes.

## Description

The present invention relates to the use of hyaluronic acid as lubricant for ocular prosthesis.

More particularly, this invention relates to the use of compositions comprising hyaluronic acid as lubricant in fitting either an ocular bulb substituting prosthesis into an ocular cavity, or a prosthesis for sub-atrophic or micro-ophtalmic bulbs of eyes.

Hyaluronic acid is a polysaccharide, which is present to a large extent in a number of human tissues, such as the connective tissue, and in biological fluids, such as the synovial fluid of the eye vitreous body (Balasz, E.A., Armand, G.:"Glycosaminoglycans and Proteoglycans in physiological and pathological processes of Body Systems, Varmas, R.Ed. Karger Basel, 1982).

Generally, hyaluronic acid is used by means of intrarticular injections, as adjuvant in the therapy of degenerative and/or inflammatory diseases of the articular synovial sheats.

Hyaluronic acid is used largely in the ophtalmic surgery, due to its elasto-viscous properties and rheologic properties (Balasz, E.A., Fed.Proc., 17, 1086-1093, 1958). Particularly, hyaluronic acid is used in cataract operations, that are associated with intraocular lens implantation, as well as glaucoma and corneal tranaplant operations and more generally in any traumatopathy involving retinal detachement.

Moreover, diluted solutions of hyaluronic acid, less than 0.2 % w/v, could be used to relieve from symptoms of lacrimal film diseases, as substitute of mucines, that are present in the physiological state (Stuart, A.C. and Linn, J.G., "Ann. of Ophtalmol.", 17 (3), 190-192, 1985).

Users of ocular prosthesis, to be fitted either into anophtalmic cavities or to sub-atrophic or micro-opthalmic bulbs of eyes, often suffer from an excess of ocular cavity dryness, particularly under special weather or environmental conditions, such as windy or cold conditions, or because of prolonged stay in too heated or dusty rooms. This is very troublesome, as it avoids the eyelids to correctly slide, thus causing conjunctive inflammatory process, due to the reaction against the eyelid friction. These problems occur frequently to patients with a prosthesis into the anophtalmic cavity, where the lacrimal gland function is very poor.

The collyrium compositions of the previous art are uneffective. Actually, the so-called "Artificial tears", though offering an immediate relief, evaporate very quickly and therefore need to be administered continuously.

Vaseline-based ointments, though having higher stability and more lasting effect, are deposited on the front surface of the prosthesis, causing a lot of aesthaetic prejudice. Moreover, vaseline, though having acceptable lubrication properties, has a poor elastoviscosity, thus causing the ointment, which is present between the prosthesis and the underlying tissues, to be gradually removed under shear conditions.

The authors of the present invention found that hyaluronic acid solutions are useful as elasto-viscous lubricants in fitting ocular prosthesis, as they are able to damp the pressure changements, frequently occuring between the ocular prosthesis and the underlying tissues.

Moreover, the authors have defined the optimized hyaluronic acid concentrations, that differ from the usual concentrations for other uses. Hyaluronic acid compositions according to this invention showed a good stability, a sufficient lasting lubrication effect and a transparent appearance. Furthermore, a decrease of the reaction conjunctive hyperaemia is experienced on treated patients, due to the anti-inflammatory action of hyaluronic acid.

Therefore, it is an object of the present invention the use of compositions comprising hyaluronic acid having the formula: 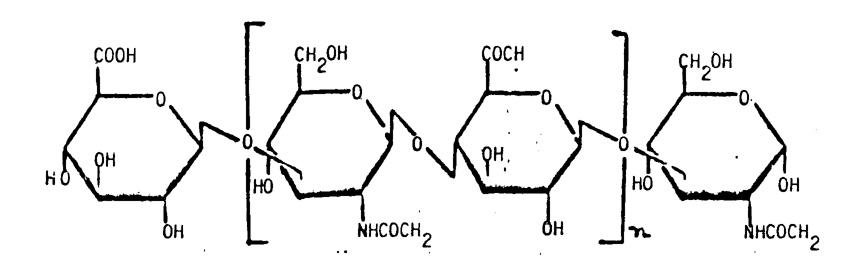
where 400 < n < 8000, for the manufacture of a medicament acting as lubricant of ocular prosthesis.

Preferably, said medicament comprises hyaluronic acid at a concentration from 0.2% to 2.0% by weight, more preferably at a concentration from 0.7% to 1.25% by weight.

According to a preferred embodiment of the invention, said compositions of hyaluronic acids comprise hyaluronic acid at purity grade higher than 99%, preferably extracted from non pathogenic bacteria of the Streptococcus genus.

Furthermore, according to the invention, said compositions comprise a NaCl solution in such amount to give the solution with a cryoscopic lowering from 0.1°C to 0.7°C, and a phosphate buffer at pH comprised between 6.5 and 7.5.

The present invention will be described with reference to an applicative but not limitating example.

### Example 1

Commercially available hyaluronic acid is purified either by cock creats or by Streptococcus non pathogenic bacterial strains, grown into fermenters. This last source produces a material of higher purity degree and with a more uniform molecular weigth.

The authors of the invention have used hyaluronic acid from bacteria fermentation, as described in US Patent n.4,480,414; however it is to be understood that any other source and/or purification procedure could be used without being out of the scope of the invention.

The used hyaluronic acid has a purity degree higher than 99% and a molecular weigth from 200,000 to 4,000,000 dalton. The purified hyaluronic acid is dissolved into a NaCl solution in such an amount to give the resulting solution with a cryoscopic lowering from 0.1°C to 0.7°C in the presence of a phosphate buffer with pH 7.0. The more effective concentration results to be of 1.0%, as related to the parameters which will be discussed in the following.

The composition, which appears as a gel, has to be administered in two phases. When the prosthesis is introduced into the ocular cavity, a gel amount of appr. 1 cm is spread over the concave surface of the prosthesis. After the prosthesis has been inserted, an other gel amount of appr. 1 cm is also administered to the internal part of the superior eyelid; by allowing the eyelid to open and close two or three times, the product is spread throughout the prosthesis surface and produces a transparent film, which lubricates the eye cavity as well as the eyelid by any winkling motion.

The results of a comparison with other products, as related to the indicated parameters, are summarized in the table 1 herebelow.

**Table 1**

| | Lubricant | | |
|---|---|---|---|
| | Artificial Tears | Vaseline-based Ointment | Hyaluronic Acid (1%) |
| Prosthesis contact time | +-- | +++ | +++ |
| Lubrication efficiency | +-- | ++- | +++ |
| Transparency | +++ | +-- | +++ |
| Anti-inflammatory effect | --- | --- | +++ |

The present invention has been described with reference to a preferred embodiment; however it is to be understood that any change and/or modification could be made by any person skilled in the art without being out of the scope of the invention.

## Claims

1. Use of compositions comprising hyaluronic acid with the formula: 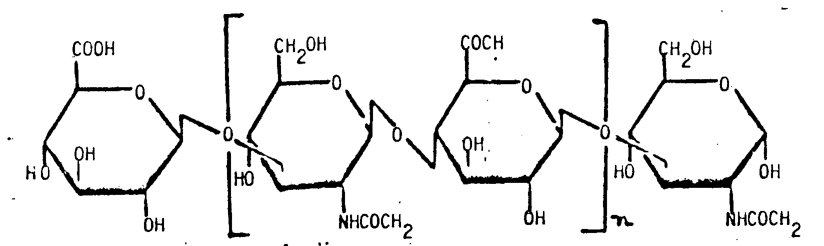 where 400 < n < 8000, for the manufacture of a medicament acting as lubricant of ocular prosthesis.

2. The use of compositions according to the claim 1, wherein said medicament comprises hyaluronic acid at a concentration from 0.2% to 2.0% by weight.

3. The use of compositions according to the claim 2, wherein said medicament comprises hyaluronic acid at a concentration from 0.7% to 1.25% by weight.

4. The use of compositions according to anyone of previous claims, wherein said compositions comprise hyaluronic acid at purity grade higher than 99%.

5. The use of compositions according to the claim 4, wherein said hyaluronic acid at purity grade higher than 99% is extracted from non pathogenic bacteria of the Streptococcus genus.

6. The use of compositions according to anyone of previous claims, wherein said medicament comprises a NaCl solution in such an amount to give the solution with a cryoscopic lowering from 0.1°C to 0.7°C in presence of a phosphate buffer with pH from 6.5 to 7.5.
